Europäisches Patentamt

European Patent Office

Office européen des brevets

(19)

(11) Publication number: **0 366 151**
**A2**

# EUROPEAN PATENT APPLICATION

(21) Application number: **89120034.7**

(22) Date of filing: **27.10.89**

(51) Int. Cl.5: **G01N 33/543 , G01N 21/17**

(30) Priority: **28.10.88 JP 270888/88**

(43) Date of publication of application:
**02.05.90 Bulletin 90/18**

(84) Designated Contracting States:
**DE GB**

(71) Applicant: **HITACHI, LTD.**
**6, Kanda Surugadai 4-chome Chiyoda-ku**
**Tokyo(JP)**

(72) Inventor: **Kitamori, Takehiko**
**1174-546, Ushiku-machi**
**Ushiku-shi Ibaraki-ken(JP)**
Inventor: **Matsui, Tetsuya**
**4-6-14-4, Suwa-cho**
**Hitachi-shi Ibaraki-ken(JP)**
Inventor: **Sakagami, Masaharu**
**419-6, Takaba**
**Katauta-shi Ibaraki-ken(JP)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) **Method and apparatus for photoacoustic immunoassay.**

(57) The present invention enables an analysis of immunoreaction products by an accurate photoacoustic spectrometry without taking into account the differences among individuals, cases and the complications of a sample.

In the present invention, a sample of an examined object is divided into two groups, sample A and sample B. Micro particles coated with an antigen or an antibody are added to sample A (the added sample is called sample A'), the micro particles not coated with the antigen or the antibody are added to sample B (the added sample is called sample B'). Photoacoustic signal magnitude detected values for both samples A' and B' are acquired and the differential signal between the detected values is made a detected value of the immunoreaction product.

FIG. 1(a)

BEFORE REACTION

AFTER REACTION

FIG. 1(b)

## Method and apparatus for photoacoustic immunoassay

### BACKGROUND OF THE INVENTION

When a ray of light with high intensity is radiated into a sample, the temperature of the sample rises by receiving the energy of the light. The temperature of the sample rises because the temperature of the medium itself rises as a result of absorbing the light energy and because the particles in the sample release the absorbed light energy in the particles as heat energy present in the medium around the particles. Thus, when the intensity of the light is modulated and radiated to the sample, the temperature of the whole medium in the area where the light is radiated and the medium around the particles in the above area rises or drops according to the degree of the modulation. An acoustic wave is generated from the whole medium and the particles around the medium accompanying the rise and drop of the temperature. As the nature of the acoustic wave differs depending on the type of the sample, that is the type of the medium and the particles, the nature of the sample, that is, of the medium and the particles, to the contrary, becomes known by measuring the temperature of the sample and the changes in degree of modulation of the radiated light.

When it is measured if a certain immunoreaction progresses or not in a sample using this photoacoustic immunoassay, the particles coated with an antigen and so forth, for example, the particles of polystyrene with a diameter of about 0. 2 are put in the sample, and the light is intensity-modulated and radiated into the sample. The acoustic wave generated caused by a temperature change is detected by, for example, a piezo-electric element. When an immunoreaction occurs and bound products are generated, a detection signal shows a particular value and the presence of the bound products, that is, the immunoreaction can be known.

Incidentally, the Japanese Patent Laid-Open No. 63-44149 is known as a conventional photoacoustic immunoassay apparatus.

For example, when a biotic substance such as a serum is analyzed by the photoacoustic immunoassay method, there are various dissolved matters, such as albumin in the sample, other than immunoreaction products. As the photoacoustic spectrometry is extremely highly sensitive, such dissolved matters are also detected and become detection noise which can obscure a detection signal of the proper immunoreaction products. Also, for example, when a hormone in urine is immunologically analyzed with the urine as a sample, the detection results of the photoacoustic spectrometry may be different if an absorbance coefficient, that is, color of the urine changes.

The above-mentioned dissolved matters like albumin or the absorbance coefficient of the urine, etc. are greatly changed depending on the difference among individuals or cases and complications of the patient. Accordingly, in order to quantitatively analyze immunoreaction products with accuracy utilizing the highly sensitive photoacoustic immunoassay, it is necessary to pay the closest attention to the noise rather than the immunoreaction products.

### SUMMARY OF THE INVENTION

The objective of the present invention is to provide a method and apparatus of a photoacoustic immunoassay which enables an accurate analysis of immunoreaction products by a photoacoustic spectrometry without being influenced by the differences among individuals, cases or complications of a sample.

This objective can be achieved by dividing a sample of an examined object into two groups, sample A and sample B, adding minute particles c which are directly or indirectly coated with an antibody of an antigen to the sample A (the added sample is called as the sample A$'$), adding the minute particles c$'$ which are not coated with the antigen or the antigen to the other sample B (added sample is called as the sample B$'$), acquiring photoacoustic signal magnitude detected values in both the samples A$'$ and B$'$, and making the differential signal between both the detected values a detected value of the immunoreaction products. In this operation, "directly coating" means that the micro particles are directly coated with the antibody or the antigen. "Indirectly coating" means that, for example, protein is coated and absorbed with the antibody or the antigen.

When the samples A$'$ and B$'$ to which the minute particles c and c$'$ of the same material and the same diameter are added and compared, the immunoreaction progresses in sample A$'$ but not in sample B$'$. Accordingly, the immunoreaction products are generated only in sample A$'$ and its bound products are made while they are not generated in sample B$'$. Concentration of the dissolved matters or the absorbance coefficient differ depending on the differences of the cases, but they are the same in samples A and B, into which the original sample was divided. Therefore, they show the same value in samples A′ and B′ except for the immunoreaction products, which differ. Thus, the differential signal of the detected values of the samples A′ and B′ is a value which depends only on the immunoreaction products.

## BRIEF DESCRIPTION OF THE DRAWINGS

Fig. 1 (a) and (b) are explanatory views of the principle of the present invention.

Fig. 2 is a block constitutional diagram of a photoacoustic immunoassay apparatus according to the first preferred embodiment of the present invention.

Fig. 3 is a graph showing the results of measuring the quantity of the fetoprotein by the photoacoustic immunoassay apparatus shown in Fig. 2.

Fig. 4 is a block constitutional diagram of the photoacoustic immunoassay apparatus according to the second preferred embodiment of the present invention. Finally,

Fig. 5 is a perspective view of a cell used in another photoacoustic immunoassay apparatus.

## DESCRIPTION OF THE PREFERRED EMBODIMENT

Hereinafter, a preferred embodiment of the present invention will be explained by reference to the drawings.

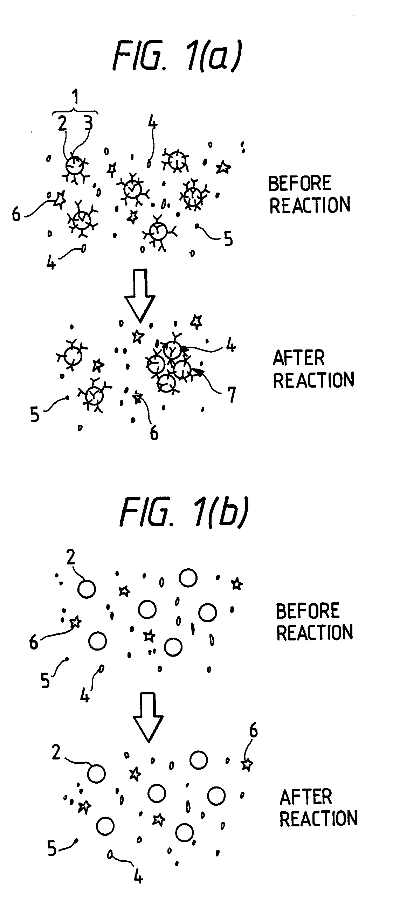

Fig. 1 (a) and (b) are the principal explanatory views of a photoacoustic immunoassay method according to a preferred embodiment of the present invention. Serum, from an examined sample, is divided into serum A and serum B. Minute particles (latex beads) (2) of synthetic resin, such as polystyrene are added to serum B to make serum B′, as shown in Fig. 1 (b). These are not coated with an antigen or an antibody. In this serum B′, there is an antigen (4), a dissolved matter (5) and a particle substance (6) besides the latex beads (2). Bound immunity products are not generated in serum B′.

The state where antigen-coated latex beads (1) coated with an antigen or an antibody are added to the other serum A to make serum A′ is shown in Fig. 1 (a). The antigen-coated latex beads (1) are made by adhering an antibody (3) to the surface of the latex beads (2). When these antigen-coated latex beads (1) are added to serum A, the antigen-coated latex beads (1) are bound through the antigen (3), thus, generating the bound products (7). The remaining dissolved matter (5) and the particle substance (6) are unchanged. Concentration of the dissolved matter (5) and the particle substance (6) vary from person to person and largely differ depending on the particular case and its complications even in the same person. Therefore, when only serum A′ shown in Fig. 1 (a) is examined by the photoacoustic spectrometry, a detection signal of the bound products (7), which exist in only small amounts, is obscured by noise generated by the presence of the dissolved matter (5) and the particle substance (6). However, the concentration of the dissolved matter 5 and the particle substance (6) in serum A′ should be the same as that in serum B′ which uses the same serum B. Therefore, when there is a difference between this detected value and the detected value of the serum A′, which is acquired with the result of serum B′ examined by the photoacoustic spectrometry as its background, only the detected value by the bound products (7) in serum A′ can be removed.

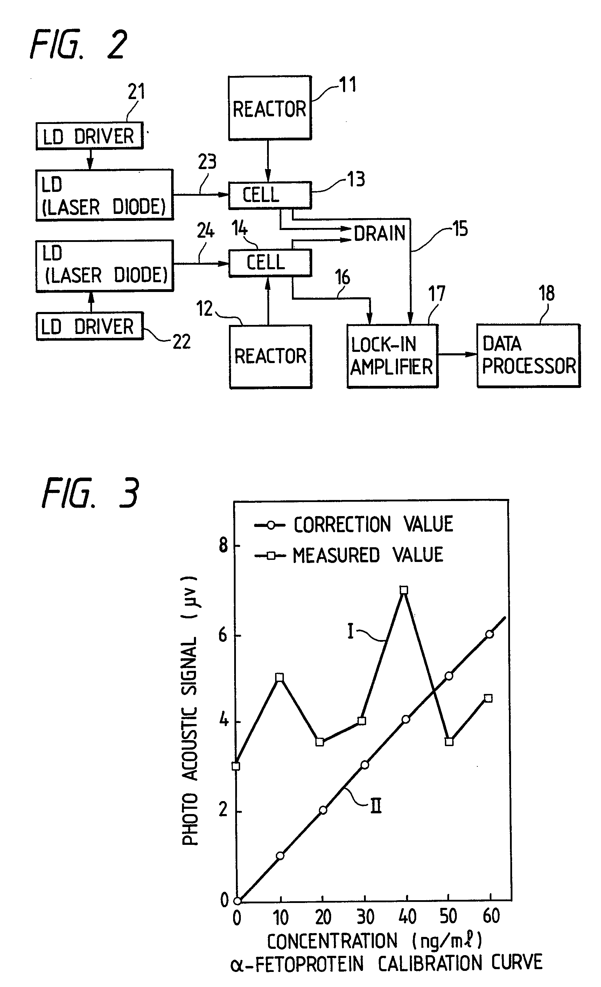

Fig. 2 is a block constitutional diagram of the photoacoustic immunoassay apparatus according to the first preferred embodiment of the present invention. The photoacoustic immunoassay apparatus is provided with two reactors (11) and (12). Cells (13) and (14), which receive the transfer of the contents, respectively from each of the reactors (11) and (12), are provided with detectors made of piezo-electric elements. Those detectors send out electric signals (15) and (16) corresponding to photoacoustic signals sent out from the contents when they receive light radiation. The detection signals (15) and (16) from each of the cells (13) and (14) are input into a lock-in amplifier (17). The lock-in amplifier (17) works by a differential input and outputs to a data processor (18) the value obtained by subtracting the magnitude of the signal (16) from the magnitude of the signal (15). The data processor (18) executes data processing such as preparing a calibration curve and calculating the antigen concentration of an unknown concentration sample based on the calibration curve. Laser diodes (19) and (20) modulate the intensity of the radiated laser beam to a rectangular wave of 180 Hz by modulating the driving electric current value from the laser diode drivers (21) and (22). These modulated excitation beams (23) and (24) are radiated to each of the cells (13) and (14). The wavelength of these intensity-modulated beams (23) and (24) is adjusted by a wavelength converter, which is not shown, so that it is equal to or approximate to, the diameter of the bound products (7) shown in Fig. 1 (a). The wavelength of the intensity-modulated beams (23) and (24) can be any wavelength, but as already mentioned, the sensitivity to the bound products (7) may be highest by substantially according it with the diameter of the bound products (7), the detected object.

The case in which the serum explained in Fig. 1 is examined using the above constituted photoacoustic immunoassay apparatus will be explained. First, the serum, the examined sample, is divided into two groups, serum A and serum B.

Serum A is put in the reactor (11) and the particles coated with an antigen and various reagents necessary for the immunoreaction are also introduced. The other serum B, as well as the particles, not coated with an antigen, of the same material and the same diameter as the antigen-coated particles introduced into the reactor (11) and the same reagent introduced into the reactor (11) in the same amount are put into the reactor (12). These two reactors (11) and (12) are kept in the same environment and the immunoreaction is subjected to identical reaction conditions. After the immunoreaction progresses, the contents of these reactors (11) and (12), that is, the reaction samples A' and B' are transferred to each of the cells (13) and (14). The intensity-modulated beams (23) and (24) are, then, radiated to each of the cells (13) and (14).

The particle substance in the reaction sample A' contained in the cell (13), the bound products (7) explained in Fig. 1, the dissolved matter (5) and the particle substance (6) absorb the radiated intensity-modulated beam (23) and generate photoacoustic signals. As the wavelength of the radiated beam (23) is set substantially equal to the size of the above bound products (7), the magnitude of the photoacoustic signal from the bound products (7) is higher than that of the photoacoustic signal from other particle substance and the magnitude is proportional to the concentration of the bound products (7). However, the concentration of the other particle substances (5) and (6) largely differ depending on the individual cases. When the concentration is high, the magnitude of the photoacoustic signals from other particle substances, the background value, becomes relatively high and varied and the photoacoustic signal form the bound products (7) is obscured by it.

However, according to this preferred embodiment, the size of this background value can be acquired by radiating the intensity-modulated beam (24) to the reaction sample B' in the cell (14). Namely, the contents in the cell (14) correspond to the contents in the cell (13) exclusive of the bound products (7) by the immunoreaction. The photoacoustic signal detected from the cell (14) corresponds to the background value among the signals detected from the cell (13). Then, a detected signal of the photoacoustic signal caused only by the bound products (7) is extracted, amplified and input to the data processor (18) by acquiring the differential signal between the detected signals of the cells (13) and (14) in the lock-in amplifier (17). In this way, reliable examination results can be obtained.

Fig. 3 is a graph showing the results of a quantitative experiment of $\alpha$-fetoprotein ($\alpha$-FT) us-

ing the abovementioned photoacoustic immunoassay apparatus. The serum of some people whose $\alpha$-FT was known was taken. $\alpha$-FT was further added to it and six samples of 10 to 60 ng/ml by 10 ng/ml were prepared. The background values of these six samples vary depending on the individual. Accordingly, the detected magnitude of the photoacoustic signal of each sample disperses as shown by line I in Fig. 3, which does not give the correct $\alpha$-FT amount. However, when the photoacoustic immunoassay apparatus in Fig. 2 is used, the background can be corrected and the amount of $\alpha$-FT can be quantitatively determined as shown by line II in Fig. 3.

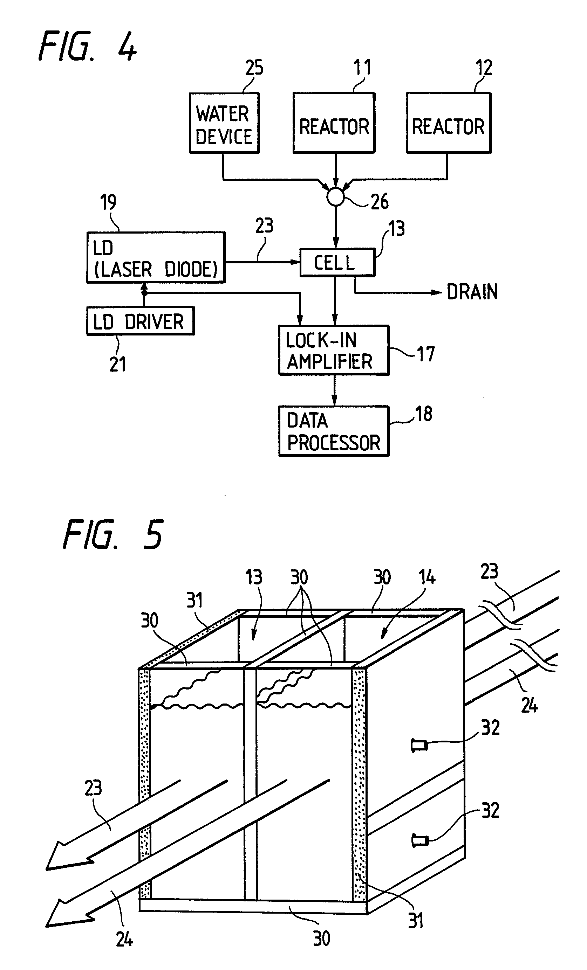

Fig. 4 is a block constitutional diagram of the second preferred embodiment of the present invention. In the photoacoustic immunoassay apparatus of the first preferred embodiment explained in Fig. 2, the photoacoustic signals from both of the cells were obtained by radiating the intensity-modulated beams simultaneously to the sample in which immunoreaction occurred and to the sample in which immunoreaction did not occur. The present preferred embodiment is provided only with the cell (13) and sample A' and B' are examined in order. That is, first, sample A' is transferred to the cell (13) by a four-way valve (26), the intensity-modulated beam (23) is radiated and magnitude of the photoacoustic signal is acquired at the lock-in amplifier (17). The data processor (18), then, stores this value in the memory. Next, the contents in the cell (13) are drained and the cell (13) is washed by introducing ultra-pure water from a tank (25) by the valve (26). After the washing, the water is drained and the reaction sample B' in the reactor (12) is introduced into the cell (13) by the valve (26). The intensity-modulated beam (23) is, then, radiated to the cell (13) again and the magnitude of the obtained photoacoustic signal is measured at the lock-in amplifier (17). This is input to the data processor (18). The data processor (18) determines the difference between the magnitude data of the photoacoustic signal of the sample A' stored in the memory and the magnitude data of the photoacoustic signal of sample B' acquired this time. This outputs a detection result based on this difference.

The same effect can also be obtained by the photoacoustic immunoassay apparatus according to this second preferred embodiment.

In the photoacoustic immunoassay apparatus according to the above-mentioned first preferred embodiment, the reactors (11) and (12) and the cells (13) and (14) are separated, but it is still possible to make the reactor (11) and the cell (13) and the reactor (12) and the cell (14) the same. For example, as shown in Fig. 5, it can be so constituted that the cell (13) and (14) are made of fused quartz 30 and piezo-electric transducers (31). Then sample A, the antigen-coated particles and the reagent are introduced into the cell (13), sample B, the antigen-coated particles and the reagent are introduced into the cell (14), react ions in both the cells (13) and (14) are left to progress under the same conditions. Finally, the intensity-modulated beams (23) and (24) are radiated to the cells (13) and (14) and electric signals appearing at electrode terminals (32) of the piezo-electric transducers are measured at the lock-in amplifier.

As mentioned above, miniaturization of the apparatus may be attempted by using the cells as reactors.

Incidentally, artificial polystyrene minute particles were used as minute particles in the above-mentioned preferred embodiment, but minute particles made of other synthetic resins or natural minute particles may also be used. In this case, a laser beam was used as the radiated light, but ordinary white light, may also be used if the intensity is great enough. Further, the present invention is not limited to the above-mentioned preferred embodiments, but includes variations within the scope of the claims described below.

The present invention, can analyze immunoreaction products with accuracy. Measured results are more reliable, even if the samples whose components differ because of the difference among individuals or cases are used as the examined samples.

**Claims**

1. A photoacoustic immunoassay method characterized in that a sample of an examined object is divided into two groups, micro particles which are not directly or indirectly coated with an antigen or an antibody are added to one sample and micro particles which are directly or indirectly ooated with the antigen or the antibody are added to the other sample. A ray of light is radiated to both samples to which the micro particles have been added and the magnitude of photoacoustic signals is detected. This difference between the detected values is made a detected value of immunoreaction product.

2. A photoacoustic immunoassay method characterized in that a sample of an examined object is divided into two groups, micro particles which are not directly or indirectly coated with antigen or an antibody are added to one sample and micro particles which are directly or indirectly coated with the antigen or the antibody are added to the other sample. The bound products are generated in the micro particles by immunoreaction. The ray of light with a wavelength which substantially accords with the size of the bound products is radiated to both samples to which the micro particles have been added and magnitude of photoacoustic signals is detected. Finally, the difference between both detected values is made a detected value of immunoreaction product.

3. A photoacoustic immunoassay method characterized in that micro particles which are not directly or indirectly coated with an antigen or antibody are added to a part of a sample of an examined object and micro particles which are directly or indirectly coated with the antigen or the antibody are added to at least a part of the remaining sample. A ray of light is, then, radiated to both samples to which the micro particles have been added and magnitude of photoacoustic signals is detected. The difference between both detected values is made a detected value of immunoreaction product.

4. A photoacoustic immunoassay apparatus characterized by a first reactor in which micro particles directly or indirectly coated with an antigen or an antibody and a part of a sample of an examined object are put together, a second reactor in which the micro particles directly or indirectly not coated with the antigen or the antibody and at least a part of the remaining sample are put together, a first cell which receives the transfer of the contents of the first reactor stores and keeps it, a second cell which receives the transfer of the contents of the second reactor stores it and keeps. it. It is also characterized by a light radiating means for radiating the ray of light to the first and second cells, a first acoustic detecting means for detecting a photoacoustic signal generated in the first cell when the ray of light is radiated to that cell, a second acoustic detecting means for detecting a photoacoustic signal generated in the second cell when the ray of light is radiated to that cell and a data processor which acquired a detected value of immunoreaction products based on the difference between the output signals of the first acoustic detecting means and the second acoustic detecting means.

5. A photoacoustic immunoassay apparatus characterized by a first reactor in which the micro particles directly or indirectly coated with an antigen or an antibody and a part of as ample of an examined object are put together, a second reactor in which the micro particles directly or indirectly not coated with the antigen or the antibody and at least a part of th remaining sample are put together, a first cell which receives the transfer of the contents of the first reactor, stores it and keeps it and puts out a detection signal corresponding to the magnitude of a photoacoustic signal put out from the contents when the ray of light is radiated, a second cell which receives the transfer of the contents of the second reactor, stores it and keeps it and puts out a detection signal corresponding to the magnitude of a photoacoustic signal put out from the contents when the ray of light is radiated, a light radiating means for radiating the ray of light to the first and second cells, and a data processor whioh processes the detection signal of the first cell with the detection signal from the second cell as a background correction value.

6. A photoacoustic immunoassay apparatus characterized by a first reactor in which the micro particles directly or indirectly coated with an antigen or an antibody and a part of a sample of an examined object are put together and bound products are generated in the micro particles by immunoreaction, a second reactor in which the micro particles directly or indirectly not coated with the antigen or the antibody and at least a part of the remaining sample are put together, a first cell which receives transfer of the contents of the first reactor, stores it and keeps it and puts out a detection signal corresponding to the magnitude of a photoacoustic signal put out from the contents when the ray of light is radiated, a second cell which receives the transfer of the contents of the second reactor, stores it and keeps it and puts out a detection signal corresponding to the magnitude of a photoacoustic signal put out from the contents when the ray of light is radiated, a light radiating means for radiating the ray of light with the wavelength which substantially accords with the size of the bound products to the first and second cells, and a data processor which processes the detection signal of the first cell with the detection signal from the second cell as a background correction value.

7. A photoacoustic immunoassay apparatus characterized by a first cell which stores and keeps micro particles directly or indirectly coated with an antigen or an antibody and a part of a sample of an examined object and puts out a detection signal corresponding to the magnitude of a photoacoustic signal put out from the contents when the ray of light is radiated, a second cell which stores and keeps the micro particles directly or indirectly not coated with the antigen or the antibody and at least a part of the remaining sample and puts out the detection signal corresponding to the magnitude of the photoacoustic signal put out from the contents when the ray of light is radiated, a light radiating means for radiating the ray of light to the first and second cells, and a data processor which processes the detection signal of the first cell with the detection signal from the second cell as a background correction value.

8. A photoacoustic immunoassay apparatus characterized by a first reactor in which the micro particles directly or indirectly coated with an antigen or an antibody and a part of a sample of an examined object are put together, a second reactor in which the micro particles directly or indirectly not coated with the antigen or the antibody and at least a part of the remaining sample are put together, a cell which receives the transfer of the contents of the first and the second reactor, stores it and keeps it and puts out a detection signal corresponding to the magnitude of a photoacoustic signal put out from the contents when the ray of light is radiated, a switching device which puts the contents of the first and the second reactors into the cell in order, a light radiating means for radiating the ray of light to the cell, and a data processor which processes the detection signal generated when the contents of the first reactor is put into the cell with the detection signal generated when the contents of the second reactor are put into the cell as a background correction value.

9. A photoacoustic immunoassay apparatus characterized by a first reactor in which the micro particles directly or indirectly coated with an antigen or an antibody and a part of a sample of an examined object are put together, a second reactor in which the micro particies directly or indirectly not coated with the antigen or the antibody and at least a part of the remaining sample are put together, a washing tank for holding the detergent, a cell which receives the transfer of the contents of the first and second reactor and the washing tank, stores it and keeps it and puts out a. detection signal corresponding to the magnitude of a photoacoustic signal put out from the contents when the ray of light is radiated, a switching device which puts the contents of the first and second reactors and the washing tank into the cell, a light radiating means for radiating the ray of light to the cell, and a data processor which processes the detection signal generated when the contents of the first reactor are put into the cell with the detection signal generated when the contents of the second reactor are put into the cell as a background correction value.

6

# FIG. 1(a)

BEFORE
REACTION

AFTER
REACTION

# FIG. 1(b)

BEFORE
REACTION

AFTER
REACTION

## FIG. 2

## FIG. 3

α-FETOPROTEIN CALIBRATION CURVE

FIG. 4

FIG. 5